(19) Europäisches Patentamt
European Patent Office
Office européen des brevets

(11) Veröffentlichungsnummer: **0 284 892 B1**

(12) # EUROPÄISCHE PATENTSCHRIFT

(45) Veröffentlichungstag der Patentschrift: **31.10.90**

(51) Int. Cl.[5]: **C07D 333/32,** A61K 31/38

(21) Anmeldenummer: **88104163.6**

(22) Anmeldetag: **16.03.88**

(54) **Neue 2-Thienyloxyessigsäurederivate, ein Verfahren zu ihrer Herstellung und diese enthaltende pharmazeutische Präparate.**

(30) Priorität: **03.04.87 AT 820/87**

(43) Veröffentlichungstag der Anmeldung: **05.10.88 Patentblatt 88/40**

(45) Bekanntmachung des Hinweises auf die Patenterteilung: **31.10.90 Patentblatt 90/44**

(84) Benannte Vertragsstaaten: **AT BE CH DE ES FR GB GR IT LI LU NL SE**

(56) Entgegenhaltungen:
**EP-A- 0 054 442**
**EP-A- 0 109 381**
**US-A- 4 602 016**

(73) Patentinhaber: **CL PHARMA AKTIENGESELLSCHAFT, St. Peter-Strasse 25, A-4021 Linz(AT)**

(72) Erfinder: **Binder, Dieter, Dr., Sieveringerstrasse 207, A-1190 Wien(AT)**
Erfinder: **Rovenszky, Franz, Dr. Dipl.-Ing., Lagerhausstrasse 5/8, A-2460 Bruck a.d. Leitha(AT)**
Erfinder: **Ferber, Hubert Peter, Dr., Ahornweg 24, A-4021 Ansfelden(AT)**

(74) Vertreter: **Kunz, Ekkehard, Dr., Chemie Holding AG Patentwesen St. Peter-Strasse 25, A-4021 Linz(AT)**

EP 0 284 892 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue 2-Thienyloxyessigsäurederivate, ein Verfahren für ihre Herstellung und pharmazeutische Präparate, die diese Substanzen enthalten.

Substanzen mit antithrombotischer Wirkung sind bereits seit längerem bekannt. So ist beispielsweise Acetylsalicylsäure (Aspirin) in hohen Dosen auch antithrombotisch wirksam. In dieser hohen Dosierung kann sie jedoch in vielen Fällen Gastritis hervorrufen. Auch sulfatierte Polysaccharide wie z.B. Heparine besitzen eine antithrombotische Wirksamkeit. Da diese Substanzen Blutungen hervorrufen können, ist ihre Anwendung in der Humanmedizin jedoch problematisch.

Es ist auch bekannt, daß Substanzen, die die Thromboxan-$A_2$-Synthese hemmen bzw. den Thromboxan-$A_2$-Rezeptor blockieren, antithrombotisch wirksam sind. Solche Substanzen sind beispielsweise in der US-PS 4,602,016 beschrieben, wo Phenoxyalkylimidazole mit antithrombotischer Wirkung geoffenbart werden. Da jedoch das pharmakologische Wirkprofil dieser Substanzen noch nicht ausgetestet ist und es daher nicht klar ist, ob sich diese Substanzen in der Humantherapie auch wirklich verwenden lassen, besteht weiterhin ein Bedarf nach neuen Verbindungen mit antithromotischer Wirkung.

Es wurde nun gefunden, daß bestimmte 2-Thienyloxyessigsäurederivate die Thromboxan-$A_2$-Rezeptoren blockieren können.

Gegenstand der Erfindung sind daher neue Derivate der 2-Thienyloxyessigsäure der allgemeinen Formel I

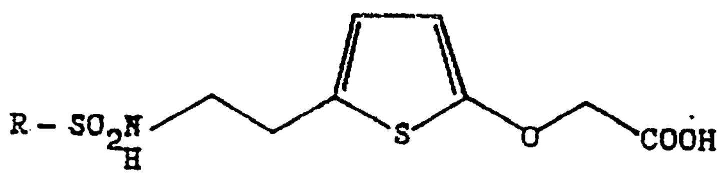

(I)

in der R eine gegebenenfalls ein- oder mehrfach durch Halogen, Trifluormethyl oder $C_1$ - $C_4$ Alkyl substituierte Phenyl- oder Thienylgruppe bedeutet, sowie deren pharmazeutisch verwendbaren Salze, ein Verfahren zu deren Herstellung, sowie pharmazeutische Präparate, die diese Verbindungen enthalten.

Unter Halogen versteht man vorzugsweise Fluor, Chlor oder Brom, insbesondere Chlor.

Der in dieser Beschreibung verwendete Ausdruck "$C_1$ - $C_4$ Alkyl" bezeichnet gesätigte geradkettige oder verzweigte Kohlenwasserstoffgruppen mit 1 bis 4 Kohlenstoffatomen, wie Methyl, Ethyl, Propyl, Isopropyl, Butyl, Isobutyl, tert. Butyl.

Von den Verbindungen der allgemeinen Formel I sind jene bevorzugt, in der R die Bedeutung von Phenyl oder 4-Chlorphenyl besitzt.

Besonders bevorzugte Einzelverbindungen sind:

5-(2-(Benzolsulfonylamino)-ethyl)-2-thienyloxyessigsäure

5-(2-(4-Chlor-benzolsulfonylamino))-ethyl)-2-thienyloxyessigsäure

Die Verbindungen der allgemeinen Formel I und deren Salze werden erfindungsgemäß dadurch hergestellt, daß man eine Verbindung der Formel II

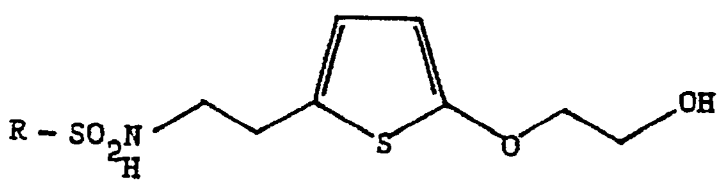

(II)

in der R die obige Bedeutung hat,

a) im wäßrigen alkalischen Medium mit Silberoxid zur Säure oxidiert und

b) gewünschtenfalls eine im Verfahrensschritt a) erhaltene freie Säure der allgemeinen Formel I mit anorganischen oder organischen Basen in ein pharmazeutisch verträgliches Salz überführt.

Die Umsetzung nach dem erfindungsgemäßen Verfahren wird am vorteilhaftesten so durchgeführt, daß man den Sulfonamidalkohol der Formel II in 0,5-4,0n, bevorzugt in 2n Alkalihydroxid-Lösung löst, mit mindestens 2 Äquivalenten $Ag_2O$ versetzt, und die Suspension unter Rühren auf eine Temperatur von etwa 75 - 85 °C erhitzt. Die Reaktionszeit beträgt etwa 2 - 3 Stunden.

Die Säuren der allgemeinen Formel I können auf übliche Weise mit anorganischen oder organischen Basen in ihre pharmazeutisch verwendbaren Salze übergeführt werden. Die Salzbildung kann beispielsweise durchgeführt werden, indem man die genannten Verbindungen der Formel I in einem geeigneten Lösungsmittel, z.B. Wasser oder einem niederen aliphatischen Alkohol, beispielsweise Methanol, Ethanol, Propanol oder Isopropanol, löst, eine äquivalente Menge der gewünschten Base zusetzt, für eine gute

Durchmischung sorgt und nach beendeter Salzbildung das Lösungsmittel im Vakuum abdestilliert. Gegebenenfalls können die Salze nach der Isolierung umkristallisiert werden.

Pharmazeutisch verwendbare Salze sind z.B. Metallsalze, insbesondere Alkalimetall- oder Erdalkalimetallsalze, wie Natrium-, Kalium-, Magnesium-oder Kalziumsalze. Andere pharmazeutisch verwendbare Salze sind beispielsweise auch leicht kristallisierende Ammoniumsalze. Letztere werden abgeleitet von Ammoniak oder organischen Aminen, z.B. Mono-, Di- oder Tri-nieder(alkyl, cycloalkyl oder hydroxyalkyl)-aminen, Niederalkylendiaminen oder (Hydroxyniederalkyl oder Aryl-niederalkyl)-niederalkylammonium Basen, wie beispielsweise Methylamin, Diethylamin, Triethylamin, Dicyclohexylamin, Triethanolamin, Ethylendiamin, Tris(hydroxymethyl)-aminomethan, Benzyl-trimethylammoniumhydroxid und dergleichen.

Die für das erfindungsgemäße Verfahren verwendeten Ausgangsverbindungen der allgemeinen Formel II können, ausgehend von bekannten Produkten, in an sich bekannter Weise hergestellt werden. Insbesondere können sie gemäß dem folgenden Reaktionsschema und den spezifischen Angaben in den Beispielen synthetisiert werden.

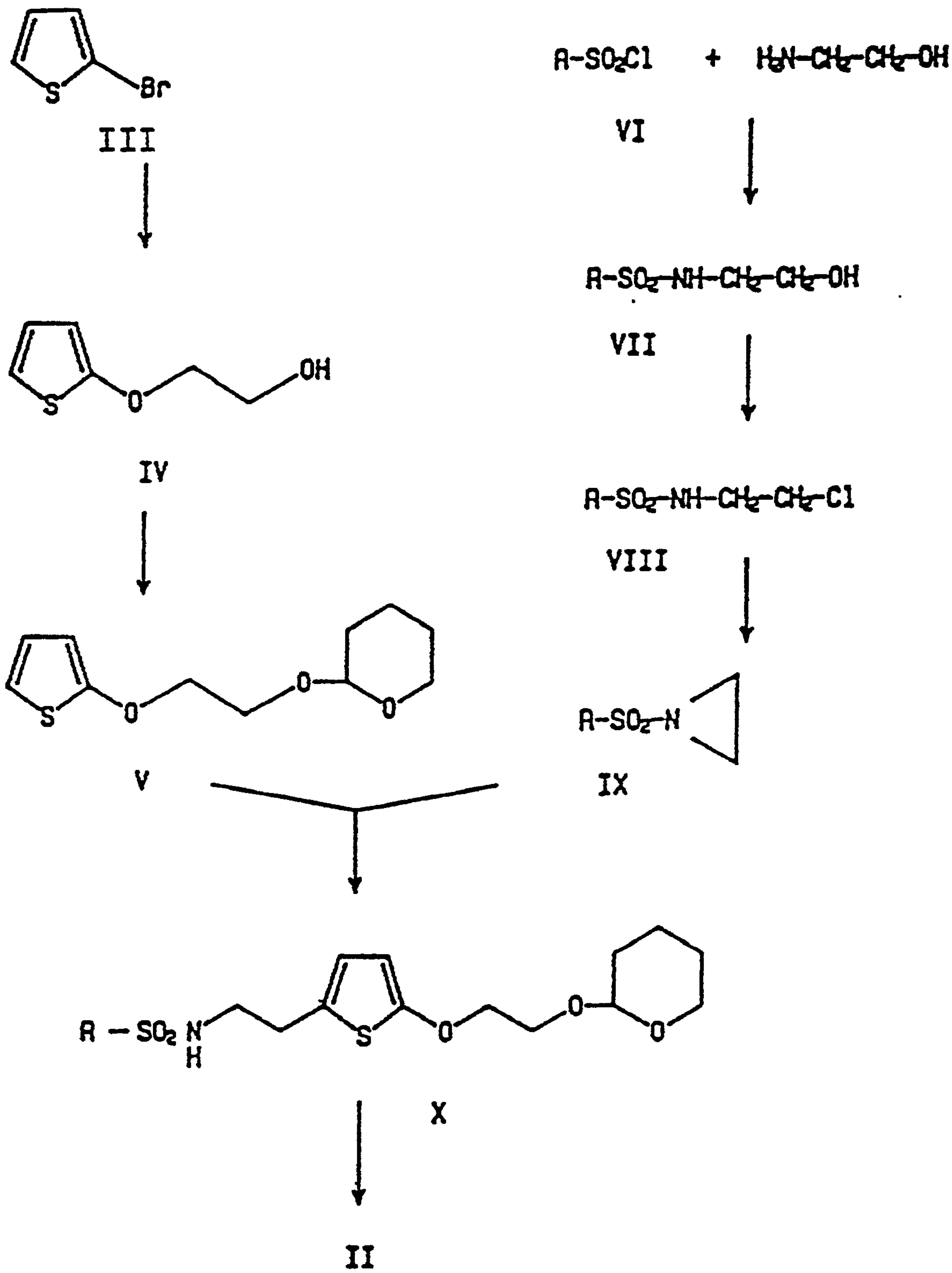

III VI VII IV VIII V IX X II

Die neuen Verbindungen der allgemeinen Formel I blockieren in vitro und in vivo die Thromboxan-$A_2$-Rezeptoren.

Aufgrund dieser pharmakologischen Eigenschaften können die neuen Verbindungen allein oder in Mischung mit anderen Wirksubstanzen in Form üblicher galenischer Zubereitungen bei Erkrankungen, die durch Thromboxan-$A_2$ verursacht werden, wie z.B. Thrombose, Entzündungen, hohem Blutdruck, Gehirnschlag, Asthma, Schock und Angina pectoris, als Medikament verwendet werden.

Zur Bestimmung der antithrombotischen Wirkung wurde die Substanz des Beispiels 1, 5-(2-(Benzolsulfonylamino)-ethyl)-2-thienyloxyessigsäure, mit Dazoxiben (4-(2-(1H-Imidazol-1-yl)ethoxy) benzoesäure-Hydrochlorid), einem in der klinischen Erprobung stehenden Antithrombotikum, wie in Beispiel A beschrieben, verglichen. Bei diesem Vergleich zeigte sich, daß die antithrombotische Wirkung der Substanz des Beispiels 1 der Wirkung von Dazoxiben deutlich überlegen ist.

Die Verbindungen der allgemeinen Formel I sind zur Verwendung am Menschen bestimmt und können auf übliche Weise, wie beispielsweise oral oder parenteral, verabreicht werden. Vorzugsweise werden sie oral verabreicht, wobei die Tagesdosis ca. 0,05 bis 20 mg/kg Körpergewicht beträgt, vorzugsweise 0,5 bis 5,0 mg/kg Körpergewicht. Der behandelnde Arzt kann jedoch, abhängig vom Allgemeinzustand und dem Alter des Patienten, der entsprechenden Substanz der Formel I, der Art der Krankheit und der Art der Formulierung, auch Dosen darüber oder darunter verschreiben.

Falls die erfindungsgemäßen Substanzen zur Prophylaxe verwendet werden, bewegen sich die Dosen ungefähr im selben Rahmen wie im Behandlungsfall. Die orale Verabreichung ist auch im Fall der Prophylaxe bevorzugt.

Die Verbindungen der Formel I können allein oder in Verbindung mit anderen pharmazeutisch aktiven Substanzen verabreicht werden, wobei der Gehalt der Verbindungen der Formel I zwischen 0,1 und 99 % liegt. Im allgemeinen liegen die pharmazeutisch aktiven Verbindungen in einer Mischung mit geeigneten inerten Hilfs- und/oder Trägerstoffen oder Verdünngungsmitteln, wie z.B. pharmazeutisch unbedenklichen Lösungsmitteln, Gelatine, Gummi arabicum, Milchzucker, Stärke, Magnesiumstearat, Talk, pflanzlichen Ölen, Polyalkylenglykol, Vaseline und dergleichen vor.

Die pharmazeutischen Präparate können in fester Form, beispielsweise als Tabletten, Dragees, Suppositorien, Kapseln und dergleichen, in halbfester Form, beispielsweise als Salben oder in flüssiger Form, beispielsweise als Lösungen, Suspensionen oder Emulsionen, vorliegen. Gegebenenfalls sind sie sterilisiert und enthalten Hilfsstofffe, wie Konservierungs-, Stabilisierungs- oder Emulgiermittel, Salz zur Veränderung des osmotischen Druckes und dergleichen. Insbesondere können pharmazeutische Präparate die erfindungsgemäßen Verbindungen in Kombination mit anderen therapeutisch wertvollen Stoffen enthalten. Mit diesen können die erfindungsgemäßen Verbindungen beispielsweise zusammen mit den oben angegebenen Hilfs- und/oder Trägerstoffen oder Verdünnungsmitteln zu Kombinationspräparaten formuliert werden.

Beispiel 1:

5-(2-(Benzolsulfonylamino)-ethyl)-2-thienyloxyessigsäure

19,3 g (0,1136 Mol) Silbernitrat werden in 120 ml dest. Wasser gelöst und langsam unter Rühren eine Lösung von 4,54 g (0,1136 Mol) Natriumhydroxid in 50 ml dest. Wasser zugetropft. Die entstandene Suspension von Silberoxid wird noch 10 Minuten gerührt, der Niederschlag abfiltriert und mehrmals mit dest. Wasser gewaschen.

9,3 g (0,028 Mol) N-(2-(2-(5-(2-Hydroxy)-ethoxy)thienyl)-ethyl)-benzolsulfonsäureamid (II) werden in 90 ml 2n wäßriger Natronlauge gelöst, das noch feuchte Silberoxid zugegeben und unter mechanischem Rühren auf 80 °C erhitzt. Nach 3 Stunden bei dieser Temperatur wird abgekühlt und die Suspension über HYFLO filtriert. Die klare Natronlaugelösung wird mit etwa 6 ml konzentrierter Salzsäure angesäuert und dreimal mit je 100 ml Ether extrahiert.

Die etherische Phase wird dreimal mit je 100 ml ges. Natriumbicarbonatlösung ausgeschüttelt, diese einmal mit 50 ml Ether gewaschen und mit konzentrierter Salzsäure angesäuert. Die wäßrige Phase wird zweimal mit 150 ml Ether extrahiert, die vereinigten Etherphasen über Natriumsulfat getrocknet, filtriert und eingedampft. Der kristalline Rückstand wird mit 30 ml Diisopropylether digeriert und abfiltriert.

Ausbeute: 2,6 g farblose Kristalle (26,4 %. d. Th.)
Schmelzpunkt: 110 - 113 °C (Ether/Diisopropylether)

$^1$H-NMR: (DMSO)
delta (ppm): 9,16 (s; breit; 1H; -COOH), 7,67 - 7,88 (m; 2H; B-$H_2$, B-$H_6$), 7,47 -7,55 (m; 3H; B-$H_3$, B-$H_4$, B-$H_5$), 7,27; 7,33; 7,47 (t; 1H; -NH-) 6,02; 6,06; 6,33; 6,38 (AB; 2H; Th-$H_3$; Th-$H_4$; $J_{34}$=3,7 Hz), 4,51 (s; 2H; O-$CH_2$-CO), 2,57 - 2,99 (m; 4H; -$CH_2$-$CH_2$-)

$^{13}$C-NMR: (DMSO)
delta (ppm): 169,1 (s; -COOH), 162,0 (s; Th-$C_2$), 140,3 (s; B-$C_1$), 131,8 (s; B-$C_4$), 128,5 (d; B-$C_3$,B-$C_5$), 127,7 (s; Th-$C_5$), 126,3 (d; B-$C_2$,B-$C_6$), 122,0 (d; Th-$C_4$), 105,3 (d; Th-$C_3$), 69,5 (t; O-$CH_2$-CO), 43,8 (t; -NH-$CH_2$-), 30,2 (t; Th-$CH_2$-).

Das Ausgangsmaterial kann wie folgt hergestellt werden:

2-(2-Thienyloxy)-ethanol

Zu 1600 ml abs. Ethylenglycol werden 323,7 ml 5,4 m Natriummethylatlösung (1,75 Mol) zugegeben. Das Reaktionsgemisch wird erwärmt und das gebildete Methanol unter Durchleiten von Stickstoff über einen Rückflußteiler abdestilliert, bis die Sumpftemperatur auf 130 °C ansteigt. Nach beendeter Methanolentfernung werden 187,5 g (1,15 Mol) 2-Bromthiophen, 55,5 g fein gemahlenes Kupferoxid und 5,6 g Natriumjodid zugegeben, die Apparatur noch kurz mit Stickstoff gespült, mit einem Ballon verschlossen und 175 Stunden bei 80 °C gerührt.
Anschließend wird das Reaktionsgemisch abgekühlt und über HYFLO abgesaugt. Das Filtrat wird mit 800 ml Wasser verdünnt und mit konzentrierter Salzsäure leicht angesäuert.
Es wird viermal mit je 400 ml Methylenchlorid (insgesamt 1600 ml) extrahiert. Die vereinigten organischen Phasen werden einmal mit 200 ml Wasser ausgeschüttelt, über Natriumsulfat getrocknet, filtriert und eingedampft. Der Rückstand wird destilliert.

Ausbeute: 102,7 g farbloses Öl (62 % d. Th.)
Kp.: 90 - 95 °C/0,6 mbar

N-(2-(2-(5-(2-Hydroxy)-ethoxy)-thienyl)-ethyl)-benzolsulfonsäureamid

30 g (0,208 Mol) 2-(2-Thienyloxy)-ethanol werden in 300 ml abs. Tetrahydrofuran vorgelegt und 50 mg p-Toluolsulfonsäure darin gelöst. Zur Lösung werden 18,37 g (0,218 Mol) 3,4-Dihydropyran zugesetzt und 8 Stunden gerührt.
Es wird auf -20 °C abgekühlt und unter Rühren 83,2 ml (0,208 Mol) 2,5m Lösung von n-Butyllithium in n-Hexan so zugetropft, daß die Temperatur nicht über - 10 °C steigt. Es wird auf Raumtemperatur erwärmen gelassen und eine Stunde gerührt.
Das Reaktionsgemisch wird auf 10 °C gekühlt und während 30 Minuten eine Lösung von 19,05 g (0,104 Mol) N-Benzolsulfonylaziridin (DRP. 698597 (1939)) in 100 ml abs. THF bei 10 - 15 °C zugetropft. Es wird auf Raumtemperatur erwärmt und noch 2 Stunden gerührt.
Das Gemisch wird auf 200 ml 2n wäßriger HCl geleert und dreimal mit je 120 ml Methylenchlorid extrahiert. Die vereinigten org. Phasen werden über Natriumsulfat getrocknet, filtriert und eingedampft. Der Rückstand wird in 300 ml abs. Methanol aufgenommen, mit 2 ml 30 %iger methanolischer Salzsäure versetzt und 10 Minuten bei Raumtemperatur gerührt. Nach Zugabe von 2 g Natriumcarbonat wird im Vakuum eingedampft.
Der Rückstand wird zwischen 250 ml 1n wäßriger Natronlauge und 200 ml Ether verteilt und die etherische Phase einmal mit 50 ml 1n Natronlauge nachgewaschen. Die vereinigten wäßrigen Phasen werden zweimal mit je 100 ml Ether gewaschen, mit ca. 25 ml konzentrierter Salzsäure angesäuert und drei Mal mit jeweils 150 ml Methylenchlorid extrahiert. Die Methylenchloridphase wird über Natriumsulfat getrocknet, mit 3 g Aktivkohle versetzt, filtriert und eingedampft.

Ausbeute: 33,3 g dunkelrotes Öl (97,8 % d.Th.), das direkt in die nächste Stufe eingesetzt wird.

Beispiel 2:

5-(2-(4-Chlor-benzolsulfonylamino)-ethyl)-2-thienyloxyessigsäure

15 g (0,088 Mol) Silbernitrat wurden in 90 ml dest. Wasser gelöst und langsam unter Rühren eine Lösung von 3,5 g (0,088 Mol) Natriumhydroxid in 45 ml dest. Wasser zugetropft. Die entstandene Suspension von Silberoxid wurde noch 10 Minuten gerührt, der Niederschlag abfiltriert und mehrmals mit dest. Wasser gewaschen.
4,0 g (0,011 Mol) 4-Chlor-N-(2-(2-(5-(2-Hydroxy)-ethoxy)-thienyl)ethyl)-benzolsulfonsäureamid (II) wurden in 40 ml 2n wäßriger Natronlauge gelöst, das noch feuchte Silberoxid zugegeben und unter mechanischem Rühren auf 80 °C erhitzt. Nach 3,5 Stunden bei dieser Temperatur wurde abgekühlt und die Suspension über HYFLO abgesaugt und mit 2n wäßriger Natronlauge nachgewaschen. Die klare Natronlaugelösung wurde mit konzentrierter Salzsäure angesäuert und dreimal mit je 80 ml Ether extrahiert.
Die etherische Phase wurde zweimal mit je 50 ml gesättigter Natriumbicarbonatlösung ausgeschüttelt, einmal mit 50 ml Ether gewaschen und mit konzentrierter Salzsäure angesäuert. Die wäßrige Phasse wurde zweimal mit 150 ml Ether extrahiert, die vereinigten Etherphasen mit Natriumsulfat getrocknet, filtriert und eingedampft. Das Rohprodukt wurde aus Toluol umkristallisiert.

Ausbeute: 1,3 g farblose Kristalle (31,7 % d.Th.)
Fp.: 125 - 127 °C (Toluol)

$^1$H-NMR: ($CDCl_3$)
delta (ppm): 7,81; 7,71; 7,51; 7,42 (AB; 4H; Bz-H; $J_{AB}$=8,0 Hz), 6,39; 6,35; 6,10; 6,06 (AB; 2H; Th-$H_3$; Th-

$H_4$; $J_{AB}$=3,8 Hz), 4,85 (t; 1H; -NH-), 4,61 (s; 2H; -O-$CH_2$-COO-), 3,13 (t; 2H; N-$CH_2$-; J=6Hz), 2,80 (t; 2H; Th-$CH_2$-; J=6Hz)

$^{13}$C-NMR: (DMSO)
delta (ppm): 169,0 (s; -COOH), 161,9 (s; Th-$C_2$), 139,3 (s; Bz-$C_1$), 137,1 (s; Bz-$C_4$), 129,1 (d; Bz-$C_3$, B-$C_5$), 128,2 (d; Bz-$C_2$, Bz-$C_6$), 127,4 (Th-$C_5$), 122,4 (d; Th-$C_4$), 105,1 (d; Th-$C_3$), 69,4 (t; O-$CH_2$-CO), 43,8 (t; -NH-$CH_2$-), 29,9 (t; Th-$CH_2$)

Das Ausgangsmaterial kann wie folgt hergestellt werden:

4-Chlor-(2-(2-(5-(2-hydroxy)-ethoxy)-thienyl)-ethyl)-benzolsulfonsäureamid

18,9 g (0,137 Mol) 2-(2-Thienyloxy)-ethanol wurden in 200 ml abs. Tetrahydrofuran vorgelegt und etwa 50 mg p-Toluolsulfonsäure darin gelöst. Es wurden 14,2 g (0,169 Mol) 3,4-Dihydropyran zugesetzt und 8 Stunden gerührt.
Es wurde auf - 20 °C abgekühlt und unter Rühren 66 ml (0,165 Mol) 2,5m Lösung von n-Butyllithium in n-Hexan zugetropft, so daß die Temperatur nicht über - 15 °C stieg. Es wurde langsam auf Raumtemperatur erwärmen gelassen und eine Stunde weitergerührt.
Jetzt wurde eine Lösung von 18 g (0,083 Mol) N-(4-Chlor-benzolsulfonyl)-aziridin (V.I. Markov u. D.A. Danileiko, Zh.Org.Khim 1973 (6), 1357) in 100 ml abs. THF bei - 5 bis 0 °C zugetropft. Das Reaktionsgemisch wurde auf Raumtemperatur erwärmt und noch 30 Minuten gerührt.
Das Reaktionsgemisch wurde auf 200 ml 2n wäßr. HCl geleert und dreimal mit je 250 ml Methylenchlorid extrahiert. Die vereinigten organischen Phasen wurden mit Natriumsulfat getrocknet, filtriert und eingedampft. Der Rückstand wurde in 100 ml ab. Methanol aufgenommen, mit 10 ml 30 %iger methanolischer Salzsäure versetzt und 10 Minuten bei Raumtemperatur gerührt. Es wurde ein Löffel Natriumkarbonat zugegeben und das Methanol abdestilliert. Der Rückstand wurde zwischen 100 ml 1n wäßriger Natronlauge und 100 ml Ether verteilt und die etherische Phase einmal mit 100 ml 1n Natronlauge nachgewaschen. Die vereinigten wäßrigen Phasen wurden zweimal mit 50 ml Ether gewaschen, mit konzentrierter Salzsäure angesäuert und dreimal mit jeweils 100 ml Methylenchlorid extrahiert. Danach wurde über Natriumsulfat getrocknet, mit Aktivkohle versetzt, filtriert und eingedampft. Es wurden 10,15 g eines zähen dunklen Öls erhalten. Dieses stark verunreinigte Rohprodukt wurde über Kieselgel 60 filtriert (180 g Kieselgel, Elutionsmittel: Ethylacetat:Petroläther).

Ausbeute: 4,5 g farblose Kristalle (15 % d.Th.)
Fp: 85 - 87 °C (Benzol)

Beispiel A:

Untersuchung der antithrombotischen Wirksamkeit

Männliche Wistarratten (SPF) mit einem Gewicht von 200 - 300 g wurden mit Pentobarbitalnatrium (60 mg/kg i.p.) narkotisiert. Dann wurde den Tieren die Substanz des Beispiels 1 ("Substanz A") bzw. Dazoxiben (4-(2-(1H-Imidazol-1-yl)ethoxy)benzoesäure-Hydrochlorid) ("Substanz B") intravenös injiziert. Eine Venole des Mesenteriums wurde freipräpariert, mit Klammern an einem Objekt tisch eines Mikroskops befestigt und mit konstant 2,5 ml/min physiologischer Kochsalzlösung bespült. Der Laserstrahl eines Coherent CR 2 supergraphite Ionenlasers (Argonlaser) wurde eine halbe Stunde nach Injektion der Testsubstanz durch das Interferenzkontrastobjektiv 50 × eines Leitz Orthoplan Mikroskopes mit einer Zeitdauer von 1/30 Sek. auf die Venole gelenkt. Die Ausgangsenergie unter dem Objektiv des Mikroskopes betrug 0,18 Watt. Wenn sich nach der esten Laserläsion (= Laserschuß) kein Plättchenthrombus bildete oder wenn der Thrombus in Länge und Breite nicht dem Gefäßdurchmesser entsprach, wurden weitere Laserschüsse gesetzt, um einen Thrombus zu erzeugen, der in Länge und Breite dem Gefäßdurchmesser entsprach.
Die Anzahl der Laserschüsse ist hiebei ein Maß für die antithrombotische Wirksamkeit der Testsubstanzen: Je größer die Anzahl der Laserschüsse bei gleichem Gefäßdurchmesser, desto stärker ist der antithrombotische Effekt.
Als Kontrollen dienten Tiere, die keine Testsubstanzen erhalten hatten.
Die Tests wurden je Testsubstanz und Konzentration an 5 Tieren durchgeführt, wobei je Tier 3 Gefäße mit einem Durchmesser zwischen 20 und 30 µm geschädigt wurden. Die statistische Auswertung erfolgte nach dem Kruskal- und Wallis-Test und nach dem Rangsummentest nach Dunn.

Ergebnisse:

Die Ergebnisse der Versuche sind in Tabelle 1 zusammengefaßt:

| Konzentration (mg/kg) | Anzahl der Subst. A | Laserschüsse (Mittelwert) Subst. B |
|---|---|---|
| 0 (Kontrolle) | 2,93 ± 0,36 | 2,93 ± 0,36 |
| 1 | 3,12 ± 0,52 | 3,07 ± 0,47 |
| 5 | 6,40 ± 0,56 | 2,89 ± 0,57 |
| 10 | 6,13 ± 0,30 | 3,14 ± 0,66 |
| 15 | 6,33 ± 0,53 | 4,46 ± 0,48 |
| 20 | 6,42 ± 0,41 | 6,35 ± 0,55 |

Diskussion:

Der Beginn der antithrombotischen Wirkung liegt bei Substanz A bei 1 mg/kg Körpergewicht und erreicht das Wirkungsmaximum bei 5 mg/kg. Eine weitere Erhöhung der Konzentration bringt keine weitere Wirkungssteigerung. Um mit Substanz B eine dem Wirkungsmaximum von Substanz A vergleichbare Wirkung zu erzielen, ist die Injektion von 20 mg/kg, also die 4-fache Menge, notwendig.

**Patentansprüche für die Vertragsstaaten: AT, BE, CH, DE, FR, GB, IT, LI, LU, NL, SE**

1. 2-Thienyloxyessigsäure-Derivate der allgemeinen Formel I

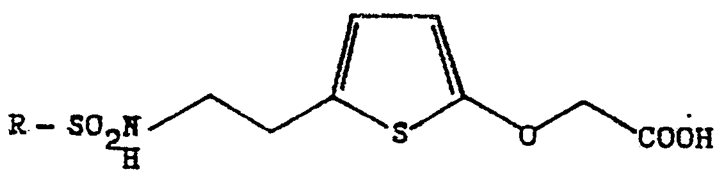

(I)

in der R eine gegebenenfalls ein- oder mehrfach durch Halogen, Trifluormethyl oder $C_1$ - $C_4$ Alkyl substituierte Phenyl- oder Thienylgruppe bedeutet, sowie deren pharmazeutisch verwendbaren Salze.

2. Verbindungen der allgemeinen Formel I nach Anspruch 1, worin R Phenyl bedeutet.

3. Verbindungen der allgemeinen Formel I nach Anspruch 1, worin R 4-Chlorphenyl bedeutet.

4. Die Verbindung nach Anspruch 1, 5-(2-(Benzolsulfonylamino)-ethyl)-2-thienyloxyessigsäure.

5. Die Verbindung nach Anspruch 1,5-(2-(4-Chlor-benzolsulfonylamino))-ethyl)-2-thienyloxyessigsäure.

6. Verfahren zur Herstellung von Verbindungen der im Anspruch 1 definierten allgemeinen Formel I und ihren Salzen, dadurch gekennzeichnet, daß man

a) eine Verbindung der Formel II

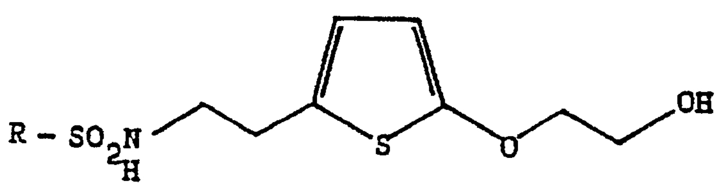

(II)

in der R wie oben definiert ist, im wäßrigen alkalischen Medium mit Silberoxid zur Säure oxidiert und

b) gewünschtenfalls eine im Verfahrensschritt a) erhaltene freie Säure der allgemeinen Formel I mit anorganischen oder organischen Basen in ein pharmazeutisch verträgliches Salz überführt.

7. Pharmazeutische Präparate, enthaltend Verbindungen der allgemeinen Formel I nach Anspruch 1 oder ein Salz davon in Kombination mit üblichen galenischen Hilfs-, Träger- und/oder Verdünngungsmitteln.

8. Pharmazeutische Präparate, enthaltend Verbindungen der allgemeinen Formel I nach Anspruch 1 oder ein Salz davon in Kombination mit anderen therapeutischen Wirkstoffen sowie üblichen galenischen Hilfs-, Träger- und/oder Verdünngungsmitteln.

9. Verbindungen nach Anspruch 1 zur Verwendung als Wirkstoffe für Arzneimittel zur Behandlung und Prophylaxe von Krankheiten, die durch Thromboxan-$A_2$ hervorgerufen werden.

**Patentansprüche für die Vertragsstaaten: ES, GR**

1. Verfahren zur Herstellung neuer 2-Thienyloxyessigsäure-Derivate der Formel I

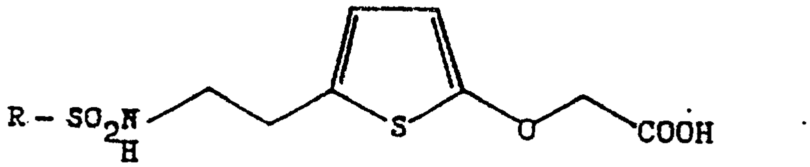

(I)

in der R eine gegebenenfalls ein-oder mehrfach durch Halogen, Trifluormethyl oder $C_1$ - $C_4$ Alkyl substituierte Phenyl-oder Thienylgruppe bedeutet, sowie von pharmazeutisch verwendbaren Salzen von Verbindungen der Formel I, dadurch gekennzeichnet, daß man

a) eine Verbindung der Formel II

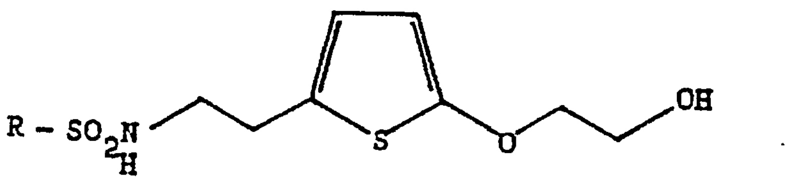

(II)

in der R wie oben definiert ist, im wäßrigen alkalischen Medium mit Silberoxid zur Säure oxidiert und

b) gewünschtenfalls eine im Verfahrensschritt a) erhaltene freie Säure der Formel I mit anorganischen oder organischen Basen in ein pharmazeutisch verträgliches Salz überführt.

2. Verfahren nach Anspruch 1, dadurch gekennzeichnet, daß man eine Verbindung der Formel II in 0,5 - 4n Alkalihydroxidlösung löst und mit mindestens 2 Äquivalenten $Ag_2O$ bei einer Temperatur von 75 - 85 °C für 2 -3 Stunden zur Säure oxidiert.

3. Verfahren nach einem der Ansprüche 1 oder 2, dadurch gekennzeichnet, daß man eine Verbindung der Formel II in 2n NaOH- oder KOH-Lösung löst.

4. Verfahren nach einem der Ansprüche 1 bis 3, dadurch gekennzeichnet, daß man das zur Oxidation benötigte $Ag_2O$ kurz vor der Oxidation durch Lösen von Silbernitrat in destilliertem Wasser und Zugabe von äquimolaren Mengen von NaOH-Lösung herstellt.

**Revendications pour les états contractants: AT, BE, CH, DE, FR, GB, IT, LI, LU, NL, SE**

1 - Dérivés de l'acide 2-thiénnyloxyactéique de formule génerale I:

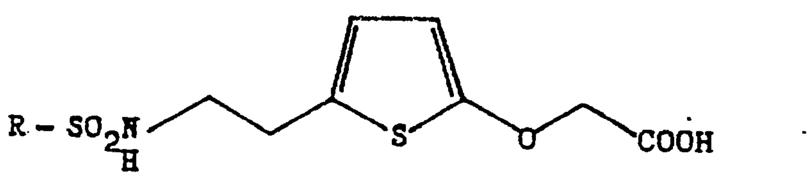

(I)

dans laquelle R est un groupe phényle ou thiényle substitué le cas échéant, une ou plusieurs fois, par un atome d'halogène, un groupe trifluorométhyle ou alkyle de 1 à 4 atomes de carbone, ainsi que leurs sels utilisables pharmaceutiquement.

2. Composé de formule générale I ou R représente un groupe phényle.

3. Composé de formule générale I ou R représente un groupe 4-chlorophényle.

4. Composé selon la revendication 1 constitué par l'acide 5-(2-(benzènesulfonylamido)-éthyl)-2-thiényl-oxyacétique.

5. Composé selon la revendication 1 constitué par l'acide 5-(2-(4-chloro-benzènesulfonylamino))-éthyl)-2-thiénylaxyacétique.

6. Procédé de fabrication de composés de formule générale I définie dans la revendication 1 et de

leurs sels, caractérisés en ce qu'on oxyde:
a) un composé de la formule II

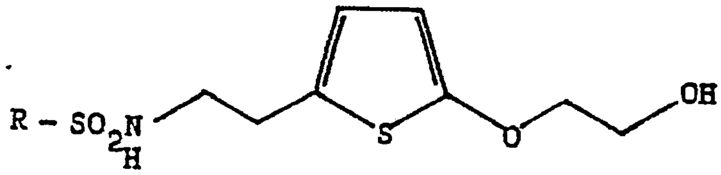

(II)

dans laquelle R est défini comme précédemment, dans un milieu aqueux alcalin avec l'oxyde d'argent, en acide, et

b) on convertit le cas échéant un acide libre de formule générale I, obtenu dans l'étape a) du procédé avec des bases organiques ou non en un sel pharmaceutiquement utilisable.

7. Préparations pharmaceutiques rentermant des composés la formule qénérale I selon la revendication 1 ou un sel en combinaison avec des adiuvants, des substances vecteurs ou agents de dilution galéniques.

8. Préparations pharmaceutiques renfermant des composés de formule générale I d' après la revendication 1 ou un sel en combinaison avec d'autres agents thérapeutiques. ainsi que des adjuvants et des substances vecteurs ou des agents diluants galéniques usuels.

9. Composés selon la revendication 1 utilisés comme principe actif dans des médicaments pour le traitement et la prophylaxie de maladies provoquées par le thromboxane-$A_2$.

**Revendications pour les états contractants: ES, GR**

1.- Procédé de préparation de nouveaux dérivés de l'acide 2-thiénylоxyacétique de formule (I)

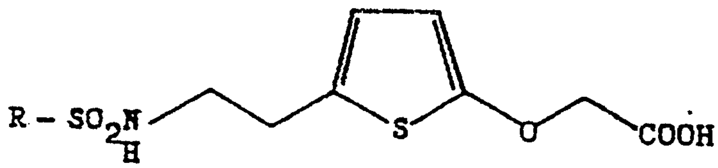

(I)

dans laquelle R signifie un groupe phényle ou thiényle substitué le cas échéant une ou plusieurs fois par un atome d'halogène, un groupe trifluorométhyle ou un groupe alkyle de 1 à 4 atomes de carbone, ainsi que les sels utilisables pharmaceutiquement des composés de formule (I), caractérisés en ce que:

(a) on oxyde un composé de formule (II)

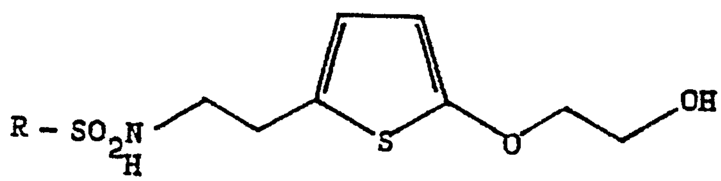

(II)

dans laquelle R est défini comme précédemment dans un milieu alcalin aqueux, avec l'oxyde d'argent en acide, et

(b) on convertit le cas échéant un acide libre de formule générale (I) obtenu dans l'étape (a) du procédé en un sel pharmaceutiquement acceptable à l'aide de bases organiques ou non.

2.- Procédé selon la revendication 1, caractérisé en ce qu'on dissout un composé de formule (II) dans une solution 0, 5 - 4 N d'hydroxyde alcalin et qu'on oxyde un acide avec au moins deux équivalents de $Ag_2O$, à une température de 75 - 85°C pendant 2 à 3 heures.

3.- Procédé selon l'une des revendications 1 ou 2, caractérisé en ce qu'on dissout un composé de formule générale (II) dans une solution 2 N de NaOH ou KOH.

4.- Procédé selon l'une des revendications 1 à 3, caractérisé en ce que l'oxyde d'argent $Ag_2O$ nécessaire à l'oxydation est préparé juste avant l'oxydation à l'aide d'une solution de nitrate d'argent dans l'eau distillée additionnée d'une quantité équimolaire de solution de NaOH.

**Claims for the Contracting States: AT, BE, CH, DE, FR, GB, IT, LI, LU, NL, SE**

1. 2-Thienyloxyacetic acid derivatives of the general formula I

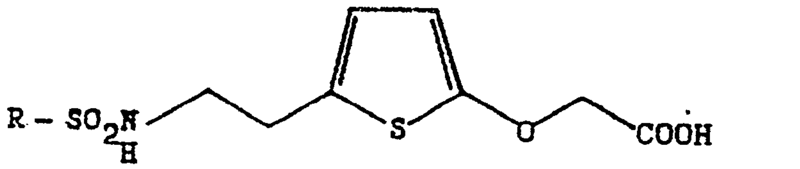

(I)

in which R denotes a phenyl or thienyl group which is optionally mono- or polysubstituted by halogen, trifluoromethyl or $C_1$-$C_4$ alkyl, and pharmaceutically usable salts thereof.

2. Compounds of the general formula I according to Claim 1, in which R denotes phenyl.

3. Compounds of the general formula I according to Claim 1, in which R denotes 4-chlorophenyl.

4. The compound 5-(2-(benzenesulphonylamino)-ethyl)2-thienyloxyacetic acid, according to Claim 1.

5. The compound 5-(2-(4-chloro-benzenesulphonylamino)-ethyl)-2-thienyloxyacetic acid, according to Claim 1.

6. Process for the preparation of compounds of the general formula I defined in Claim 1 and of salts thereof, characterized in that

a) a compound of the formula II

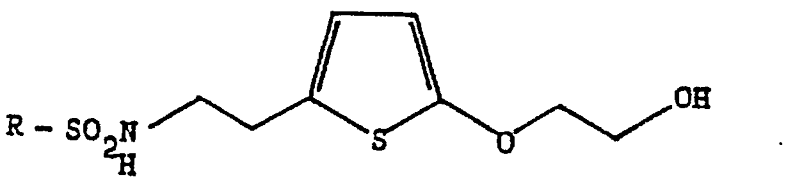

(II)

in which R has the above meaning, is oxidized with silver oxide in an aqueous alkaline medium to give the acid, and

b) if desired converting a free acid of the general formula I, obtained in process step a), into a pharmaceutically tolerated salt using inorganic or organic bases.

7. Pharmaceutical preparations containing compounds of the general formula I according to Claim 1 or a salt thereof in combination with customary galenical auxiliaries, excipients and/or diluents.

8. Pharmaceutical preparations containing compounds of the general formula I according to Claim 1 or a salt thereof in combination with other therapeutic active compounds and customary galenical auxiliaries, excipients and/or diluents.

9. Compounds according to Claim 1 for use as active substances for medicaments for the treatment and prophylaxis of diseases caused by thromboxane $A_2$.

**Claims for the Contracting States: ES, GR**

1. Process for the preparation of novel 2-thienyloxyacetic acid derivatives of the general formula I

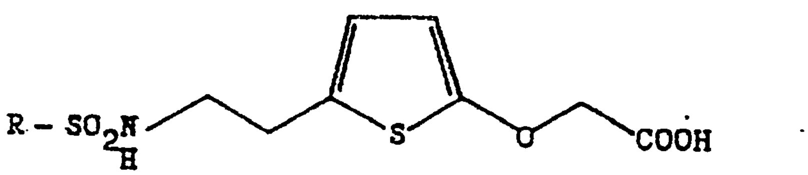

(I)

in which R denotes a phenyl or thienyl group which is optionally mono- or polysubstituted by halogen, trifluoromethyl or $C_1$-$C_4$ alkyl, and of pharmaceutically usable salts of compounds of the formula I, characterized in that

a) a compound of the formula II

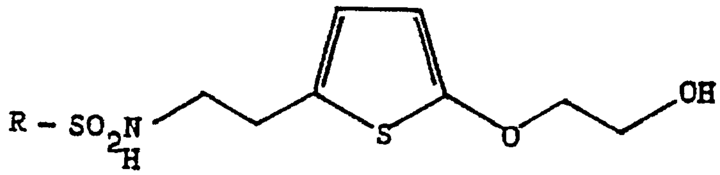

(II)

in which R has the above meaning, is oxidized with silver oxide in an aqueous alkaline medium to give the acid, and

b) if desired converting a free acid of the formula I, obtained in process step a), into a pharmaceutically tolerated salt using inorganic or organic bases.

2. Process according to Claim 1, characterized in that a compound of the formula II is dissolved in 0.5–4n alkali metal hydroxide solution and oxidized with at least 2 equivalents of $Ag_2O$ at a temperature of 75-85°C for 2-3 hours to give the acid.

3. Process according to either of Claims 1 or 2, characterized in that a compound of the formula II is dissolved in 2n NaOH or KOH solution.

4. Process according to one of Claims 1 to 3, characterized in that the $Ag_2O$ necessary for oxidation is prepared shortly before the oxidation by dissolving silver nitrate in distilled water and adding equimolar amounts of NaOH solution.